**Europäisches Patentamt**

**European Patent Office**

**Office européen des brevets**

(19)

(11) Veröffentlichungsnummer: **0 011 222**
**B1**

(12)

# EUROPÄISCHE PATENTSCHRIFT

(45) Veröffentlichungstag der Patentschrift:
**20.01.82**

(21) Anmeldenummer: **79104350.8**

(22) Anmeldetag: **07.11.79**

(51) Int. Cl.³: **B 01 J 23/24,** C 07 C 5/48,
C 07 C 15/46

(54) **Trägerkatalysator, Verfahren zu seiner Herstellung und seine Verwendung zur Herstellung von Styrol.**

(30) Priorität: **16.11.78 DE 2849637**

(43) Veröffentlichungstag der Anmeldung:
**28.05.80 Patentblatt 80/11**

(45) Bekanntmachung des Hinweises auf die Patenterteilung:
**20.01.82 Patentblatt 82/3**

(84) Benannte Vertragsstaaten:
**BE DE FR GB IT NL**

(56) Entgegenhaltungen:
**DE-A-2 626 887**
**DE-A-2 644 107**
**DE-A-2 816 946**
**FR-A-2 263 819**
**FR-A-2 291 179**

(73) Patentinhaber: **HOECHST AKTIENGESELLSCHAFT,**
**Postfach 80 03 20, D-6230 Frankfurt/Main 80 (DE)**

(72) Erfinder: **Erpenbach, Heinz, Dr., Oberbuschweg 22,**
**D-5000 Köln 50 (DE)**
Erfinder: **Gehrmann, Klaus, Dr.,**
**Geschwister-Scholl-Strasse 32, D-5042 Erftstadt (DE)**
Erfinder: **Joest, Herbert, Heddinghovener Strasse 3,**
**D-5042 Erftstadt (DE)**

## Trägerkatalysator, Verfahren zu seiner Herstellung und seine Verwendung zur Herstellung von Styrol

Die Erfindung betrifft einen Trägerkatalysator, ein Verfahren zu seiner Herstellung sowie seine Verwendung zur Herstellung von Styrol durch oxidative Dehydrierung von Ethylbenzol mit molekularem Sauerstoff in der Gasphase.

Es sind bereits Trägerkatalysatoren für die genannte Umsetzung bekannt geworden. So beschreibt die GB-PS Nr. 1148108 die oxidative Dehydrierung von Ethylbenzol mit Luft in Gegenwart von Wasserdampf bei 350 bis 600°C unter Einsatz eines Fliessbettkatalysators, bestehend aus Chromoxid und Alkalimetalloxid auf einem Trägermaterial. Gemäss US-PS Nr. 3917732 wird die oxidative Dehydrierung in Gegenwart von Magnesiumnickelpyrophosphat als Katalysator und von Helium, Stickstoff oder Wasserdampf als inertem Verdünnungsmittel durchgeführt. Die US-PS Nr. 3923916 beschreibt die oxidative Dehydrierung in Gegenwart von Nickelpyrophosphat als Katalysator und von Helium als inertem Verdünnungsmittel. Die US-PS Nr. 3935126 betrifft die genannte oxidative Dehydrierung in Gegenwart eines Erdalkalinickelphosphats als Katalysator und von Stickstoff oder Helium als inertem Verdünnungsmittel. Die US-PS Nr. 3957897 beschreibt dieselbe Umsetzung mit einem Erdalkalipyrophosphatkatalysator und Helium als inertem Verdünnungsmittel. Die meisten der bekannten Katalysatorsysteme sind für die grosstechnische Herstellung von Styrol wenig geeignet, da sie den Einsatz von inerten Verdünnungsmitteln wie Helium oder Stickstoff erfordern, die entweder eine energieaufwendige Kreislaufführung nach sich ziehen oder infolge Tiefkühlung bzw. Wäsche die Gewinnung des gebildeten Styrols aus den Inertgasen sehr erschweren. Vor allem aber erlauben die bekannten Katalysatoren bei der erforderlichen hohen Selektivität nur eine unzureichende Produktivität von höchstens 135 g Styrol je Liter Katalysator und Stunde, die überdies nur in kleinsten Apparateeinheiten aufgrund gaschromatographischer Analyse des Gasstromes festgestellt werden kann, während bei der technischen Gewinnung des Styrols gemäss GB-PS Nr. 1148108 eine Produktivität von lediglich 75 g Styrol/l · h errechenbar ist.

Aufgabe der vorliegenden Erfindung ist die Vermeidung dieser Nachteile durch Herstellung geeigneter oxidischer Trägerkatalysatoren, die eine technisch durchführbare und zugleich wirtschaftliche oxydative Dehydrierung von Ethylbenzol zu Styrol in der Gasphase ermöglichen.

Die Erfindung betrifft nun einen Trägerkatalysator aus Oxiden des Chroms und Wolframs sowie mindestens einem der Oxide des Molybdäns und des Kaliums im Atomverhältnis
$Cr_1 W_{0,1-0,5} Mo_{0-0,05} K_{0-0,5}$
auf einem porösen Trägermaterial.

Der Trägerkatalysator der Erfindung kann weiterhin bevorzugt und wahlweise dadurch gekennzeichnet sein, dass
a) das Trägermaterial eine BET-Oberfläche von 0,1 bis 500, vorzugsweise 2 bis 200 m²/g, besitzt;
b) das Trägermaterial eine Korngrösse von 0,01 bis 6 mm, vorzugsweise von 0,01 bis 0,2 mm für den Einsatz im Fliessbett oder von 3-6 mm für den Einsatz im Festbett, aufweist;
c) er 2 bis 30 Gew.% der Oxide des Chroms und Wolframs sowie des Molybdäns und/oder Kaliums enthält und
d) er Kieselsäure oder Aluminiumoxid als poröses Trägermaterial enthält.

Die Erfindung betrifft ebenso ein Verfahren zur Herstellung des Trägerkatalysators, welches dadurch gekennzeichnet ist, dass man
a) ein trockenes, poröses Trägermaterial bis zum Erreichen von 40 bis 80% des vorher ermittelten Sättigungswertes mit Wasser tränkt;
b) das vorgetränkte Trägermaterial nacheinander, jedoch in beliebiger Reihenfolge, mit einer ersten Lösung von wasserlöslichen Verbindungen des Chroms und gegebenenfalls des Kaliums in Wasser, einer zweiten Lösung einer wasserlöslichen Verbindung des Wolframs in Wasser und gegebenenfalls einer dritten Lösung einer wasserlöslichen Verbindung des Molybdäns in Wasser imprägniert, wobei jede Lösung mindestens einmal in einer höchstens zur vollständigen Sättigung des Trägermaterials ausreichenden Menge eingesetzt wird;
c) das Trägermaterial nach jeder Imprägnierung 2 bis 20 h bei 350 bis 500 K trocknet und
d) abschliessend 0,5 bis 12 h bei 550 bis 1000 K, vorzugsweise bei 600 bis 900 K im Luftstrom sintert.

Das vorgetränkte Trägermaterial wird dabei vorzugsweise bei Temperaturen von 290 bis 375 K imprägniert. Die Imprägnierung kann auch kontinuierlich unter teilweisem Eindampfen durchgeführt werden.

Schliesslich betrifft die Erfindung auch die Verwendung des Trägerkatalysators zur Herstellung von Styrol durch oxidative Dehydrierung von Ethylbenzol mit molekularem Sauerstoff in der Gasphase sowie das auf diese Weise hergestellte Styrol.

Die Erfindung ermöglicht die genannte Umsetzung mit gleichzeitig hoher Selektivität und Produktivität. Die folgenden Begriffe seien hiermit definiert:

Umsatz (%) =

$$\frac{\text{Mol eingesetztes Ethylbenzol/h} - \text{Mol aus Reaktionsprodukt rückgewonnenes Ethylbenzol/h}}{\text{Mol eingesetztes Ethylbenzol/h}} \cdot 100$$

Ausbeute (%) =

$$\frac{\text{Mol gewonnenes Styrol/h}}{\text{Mol eingesetztes Ethylbenzol/h}} \cdot 100$$

$$\text{Selektivität (\%)} = \frac{\text{Ausbeute}}{\text{Umsatz}} \cdot 100$$

$$\text{Produktivität} = \frac{\text{g gewonnenes Styrol}}{\text{l Katalysator} \cdot \text{h}}$$

Bei der Herstellung des erfindungsgemässen Katalysators setzt man Chrom und Kalium bevorzugt als Nitrate, Oxide bzw. Hydroxyde, aber auch als Chloride, Carbonate und organische Säuresalze (Formiate, Acetate, Citrate) ein. Molybdän und Wolfram werden zweckmässig in Form von Ammoniumsalzen, besonders Ammoniumheptamolybdat bzw. Ammoniumparawolframat, oder zusammen mit Kalium als Kaliummolybdat bzw. Kaliumwolframat in Lösung gebracht.

Bei der Herstellung des erfindungsgemässen Katalysators wird die Tränkung des vorzugsweise kugelförmigen $SiO_2$- oder $Al_2O_3$-Trägermaterials mit Wasser bis zum Erreichen von 40 bis 80% seines Aufnahmevermögens vorgenommen, damit die anschliessend gemeinsam bzw. einzeln nach und nach aufgetragenen gelösten aktiven Verbindungen überwiegend an der Oberfläche des Trägers angereichert werden.

Die oxidative Dehydrierung von Ethylbenzol zu Styrol kann in Gegenwart des Trägerkatalysators der Erfindung wie folgt durchgeführt werden:

Ethylbenzol und Sauerstoff werden im Molverhältnis von 1 zu 0,1 bis 1 in Gegenwart von 1 bis Mol Wasserdampf und 0 bis 4 Mol Inertgas wie $N_2$ oder $CO_2$ je Mol Ethylbenzol bei Temperaturen von 550 bis 1000 K und 1 bis 5 bar Druck durch oder über den Trägerkatalysator der Erfindung geleitet. Hierbei kann im Fest-, Fliess- oder Wirbelbett gearbeitet werden.

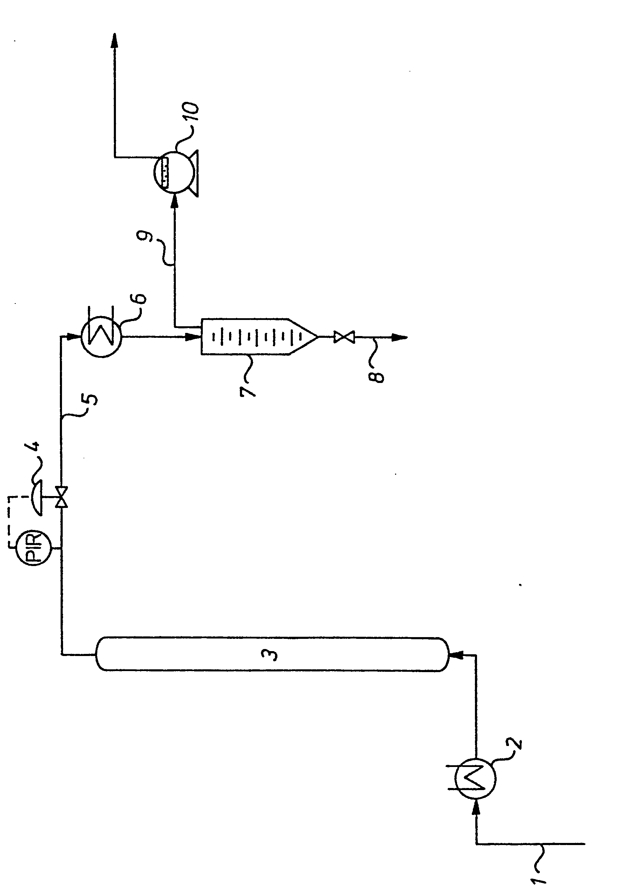

Ein mögliches Fliessschema zur Durchführung des Verfahrens ist in der Zeichnung dargestellt und im folgenden beschrieben:

Ethylbenzol, reiner Sauerstoff oder Luft, Wasserdampf und ggf. Stickstoff oder Kohlendioxid werden über Leitung (1) und den Wärmetauscher (2) dem ummantelten Reaktor (3) zugeführt. Im Wärmetauscher (2) wird die Ausgangsmischung auf 400 bis 550 K vorgeheizt. Die gewünschte Reaktionstemperatur von 550 bis 1000 K, vorzugsweise 600 bis 900 K, wird mittels eines im Mantelraum befindlichen elektrisch beheizten Sand- oder Salzbades eingestellt. Der Reaktionsdruck wird über ein automatisches Regelventil (4) auf 1 bis 5, vorzugsweise 1 bis 3 bar gehalten. Die Belastung des Katalysators mit gasförmiger Ausgangsmischung erfolgt mit einer Raumgeschwindigkeit von 100 bis 3000, vorzugsweise 500 bis 1500 $h^{-1}$ Raumgeschwindigkeit = Nl Ausgangsmischung, dividiert durch Liter Katalysator im Reaktor · h). Hieraus ergibt sich eine Verweilzeit der Ausgangsmischung am Katalysator von 0,1 bis 30 s, vorzugsweise 0,5 bis 6 s. Die Reaktionsgase verlassen den Reaktor (3) über Leitung (5) und den Kondensator (6) und trennen sich im Abscheider (7) in ein flüssiges Kondensat und Abgas. Die kondensierten Reaktionsprodukte werden über die Leitung (8) abgezogen, gewichtsmässig erfasst und gaschromatographisch analysiert. Das Abgas verlässt das System über die Leitung (9). Die Abgasmenge wird durch die Gasuhr (10) erfasst und seine Zusammensetzung gaschromatographisch ermittelt. Das erfindungsgemässe Verfahren wird durch die nachstehenden Beispiele erläutert.

*Beispiel 1:*

600 g $SiO_2$-Kugeln (Durchmesser: 4-5 mm; BET-Oberfläche: 135 m²/g) werden in einem Rotationsverdampfer mit 300 g Wasser entsprechend 67% des Sättigungswertes vorgetränkt, bevor eine Lösung von 900 g Wasser und 40 g $(NH_4)_{10}W_{12}O_{41} \cdot 11H_2O$ bei einer Temperatur von 370 K kontinuierlich unter teilweiser Verdampfung von Wasser aufgetragen wird. Nach 12stündiger Trocknung bei 390 K erfolgt die Auftragung einer Lösung von 450 g Wasser, 150,3 g $CrO_3$ und 12,74 g KOH. Nach der Imprägnierung wird abschliessend 16 h bei 400 K getrocknet und der Katalysator 10 h bei 720 K im Luftstrom gesintert. Der Katalysator enthält 24,6 Gew.% katalytisch aktive Masse der Zusammensetzung $Cr_1W_{0,1}K_{0,15}O_{3,375}$.

*Beispiel 2:*

450 g $SiO_2$ (Korngrösse 0,01 bis 0,2 mm; BET-Oberfläche 185 m²/g) werden in einem Knetbeutel mit 350 g Wasser entsprechend 58% des Sättigungswertes vorgetränkt und anschliessend mit einer Lösung von 250 g Wasser, 91,76 g $CrO_3$ und 7,72 g KOH bei einer Temperatur von 295 K verknetet. Nach 10stündiger Trocknung bei 380 K werden in einer zweiten Auftragung 1,62 g $(NH_4)_6Mo_7O_{24} \cdot 4H_2O$ in 250 g Wasser gelöst zugeführt. Nach 2 h Trocknung bei 400 K erfolgt die Auftragung einer Lösung von 1200 g Wasser und 24,77 g $(NH_4)_{10}W_{12}O_{41} \cdot 11H_2O$ in zwei Portionen mit zwischengeschalteter 10stündiger Trocknung bei 390 K. Der so hergestellte Katalysator wird 16 h bei 400 K getrocknet und 1 h bei 820 K im Luftstrom gesintert. Der Gehalt an katalytisch aktiver Masse beträgt 21,2 Gew.% in der Zusammensetzung $Cr_1W_{0,1}Mo_{0,01}K_{0,15}O_{3,405}$.

*Beispiel 3:*

552 g $Al_2O_3$ (Korngrösse 3 bis 6 mm; BET-Oberfläche 175 m²/g) werden im Rotationsverdampfer mit 400 g Wasser entsprechend 66% des Sättigungswertes vorgetränkt und mit einer Lösung von 200 g Wasser, 137,4 g $CrO_3$ und 11,44 g KOH bei 370 K unter kontinuierlicher Verdampfung von Wasser belegt. Nach 4stündiger Trocknung bei 390 K erfolgt in zwei Auftragungen das Aufbringen der zweiten Lösung von 800 g Wasser und 42,64 g $(NH_4)_{10}W_{12}O_{41} \cdot 11H_2O$. Der fertige Katalysator enthält nach 16stündiger Trocknung bei 400 K und 10stündiger Sinterung bei 720 K im Luftstrom 25 Gew.% katalytisch aktive Masse der Zusammensetzung $Cr_1W_{0,12}K_{0,15}O_{3,435}$.

*Beispiel 4:*

431 g $SiO_2$ der Korngrösse 0,01 bis 0,2 mm (BET-Oberfläche 145 m²/g) werden in einem Plastikbeutel mit 350 g Wasser entsprechend 78% des Sättigungswertes vorgetränkt und anschliessend mit einer Lösung von 100 g Wasser, 21,36 g $CrO_3$ und 1,8 g KOH bei 295 K verknetet. Nach Trocknung über 2 h bei 400 K erfolgt die Auftragung einer Lösung von 350 g Wasser und 5,77 g $(NH_4)_{10}W_{12}O_{41} \cdot 11H_2O$. Nach Trocknung über

16 h bei 400 K und Sinterung bei 670 K über 10 h im Luftstrom enthält der fertige Katalysator 6 Gew.% katalytisch aktive Masse der Zusammensetzung $Cr_1W_{0,1}K_{0,15}O_{3,375}$.

*Beispiel 5:*

600 g $SiO_2$-Kugeln (Korngrösse 5 mm; BET-Oberfläche 40 m²/g) werden in einem Rotationsverdampfer mit 500 g Wasser entsprechend 71% des Sättigungswertes vorgetränkt, bevor eine Lösung von 1200 g Wasser und 60 g $(NH_4)_{10}W_{12}O_{41} \cdot 11H_2O$ bei einer Temperatur von 370 K kontinuierlich unter teilweiser Verdampfung von Wasser aufgetragen wird. Nach 6stündiger Trocknung bei 390 K erfolgt die Auftragung einer Lösung von 500 g Wasser und 150,3 g $CrO_3$. Es wird 5 h bei 390 getrocknet und eine Lösung von 200 g Wasser und 4 g $(NH_4)_6Mo_7O_{24} \cdot 4H_2O$ aufgetragen. Nach der Imprägnierung wird abschliessend 12 h bei 400 K getrocknet und der Katalysator 10 h bei 750 K im Luftstrom gesintert. Der Katalysator enthält 25,5 Gew.% katalytisch aktive Masse der Zusammensetzung $Cr_1W_{0,15}Mo_{0,015}O_{3,495}$.

*Beispiel 6:*

658 g (1 l) Katalysator $Cr_1W_{0,1}K_{0,15}O_{3,375}$ auf $SiO_2$ als Träger gemäss Beispiel 1 werden im Festbettreaktor (1,2 l Inhalt, 121 cm Füllhöhe) stündlich mit 990 Nl (Raumgeschwindigkeit 990 h⁻¹) eines Gemisches von 13,2 Vol.% Ethylbenzol, 30 Vol.% Luft und 56,8 Vol.% Wasserdampf beschickt. Bei einem Reaktordruck von 1,05 bar, einer Reaktionstemperatur von 791 K sowie einer Verweilzeit von 1,3 s werden 32,8% des Ethylbenzols umgesetzt. Die Ausbeute an Styrol beträgt 28% und die Selektivität 85,6%. Es ergibt sich eine Produktivität von 170 g Styrol/l Katalysator · h.

*Beispiel 7:*

530 g (0,9 l) Katalysator
$Cr_1W_{0,1}Mo_{0,01}K_{0,15}O_{3,405}$
auf $SiO_2$ als Träger gemäss Beispiel 2 werden im Wirbelbettreaktor (2 l Reaktionsraum, 50 cm Füllhöhe) stündlich mit 835 Nl (Raumgeschwindigkeit 928 h⁻¹) eines Gemisches von 13 Vol.% Ethylbenzol, 30,4 Vol.% Luft und 56,6 Vol.% Wasserdampf beschickt. Bei einem Reaktordruck von 1,1 bar, einer Reaktionstemperatur von 824 K sowie einer Verweilzeit von 1,4 s werden 42,4% des Ethylbenzols umgesetzt. Die Ausbeute an Styrol beträgt 37,1% und die Selektivität 87,4%. Die Produktivität beträgt 207 g Styrol/l Katalysator · h.

*Beispiel 8:*

658 g (0,85 l) Katalysator $Cr_1W_{0,12}K_{0,15}O_{3,435}$ auf $Al_2O_3$ als Träger gemäss Beispiel 3 werden im Festbettreaktor stündlich mit 1070 Nl (Raumgeschwindigkeit 1260 h⁻¹) eines Gemisches von 11,8 Vol.% Ethylbenzol, 30,9 Vol.% Luft und 57,3 Vol.% Wasserdampf beschickt. Bei einem Reaktordruck von 2,5 bar, einer Reaktionstemperatur von 682 K sowie einer Verweilzeit von 2,75 s werden 31,3% des Ethylbenzols umgesetzt. Die Ausbeute an Styrol beträgt 27,3% und die Selektivität 86,9%. Die Produktivität beläuft sich auf 188 g Styrol/l Katalysator · h.

*Beispiel 9:*

354 g (0,9 l) Katalysator $Cr_1W_{0,1}K_{0,15}O_{3,375}$ auf $SiO_2$ als Träger gemäss Beispiel 4 werden im Wirbelbettreaktor stündlich mit 1355 Nl (Raumgeschwindigkeit 1500 h⁻¹) eines Gemisches von 15,7 Vol.% Ethylbenzol, 33,4 Vol.% Luft und 50,9 Vol.% Wasserdampf beschickt. Bei einem Reaktordruck von 1,1 bar, einer Reaktionstemperatur von 746 K sowie 0,9 s Verweilzeit werden 29,5% des Ethylbenzols umgesetzt. Die Ausbeute an Styrol beträgt 25% und die Selektivität 84,6%. Es resultiert eine Produktivität von 300 g Styrol/l Katalysator · h.

*Beispiel 10:*

520 g (1 l) Katalysator $Cr_1W_{0,15}Mo_{0,015}O_{3,495}$ auf $SiO_2$ als Träger gemäss Beispiel 5 werden im Festbettreaktor mit 1200 Nl/h (Raumgeschwindigkeit 1200 h⁻¹) eines Gemisches von 12,5 Vol.% Ethylbenzol, 31 Vol.% Luft und 56,5 Vol.% Wasserdampf beschickt. Bei einem Reaktordruck von 1,2 bar, einer Reaktionstemperatur von 725 K sowie einer Verweilzeit von 1,4 s werden 37,8% des Ethylbenzols umgesetzt. Die Ausbeute an Styrol beträgt 31,3% und die Selektivität 82,9%. Die Produktivität liegt bei 218 g Styrol/l Katalysator · h.

**Patentansprüche**

1. Trägerkatalysator aus Oxiden des Chroms und Wolframs sowie mindestens einem der Oxide des Molybdäns und des Kaliums im Atomverhältnis $Cr_1W_{0,1-0,5}Mo_{0-0,05}K_{0-0,5}$ auf einem porösen Trägermaterial.

2. Trägerkatalysator nach Anspruch 1, dadurch gekennteichnet, dass das Trägermaterial eine BET-Oberfläche von 0,1 bis 500, vorzugsweise 2 bis 200 m²/g, besitzt.

3. Trägerkatalysator nach Anspruch 1 oder 2, dadruch gekennzeichnet, dass das Trägermaterial eine Korngrösse von 0,01 bis 6 mm, vorzugsweise von 0,01 bis 0,2 mm für den Einsatz im Fliessbett oder von 3 bis 6 mm für den Einsatz im Festbett, aufweist.

4. Trägerkatalysator nach einem der Ansprüche 1 bis 3, dadurch gekennzeichnet, dass er 2 bis 30 Gew.% der Oxide des Chroms und Wolframs sowie des Molybdäns und/oder Kaliums enthält.

5. Trägerkatalysator nach einem des Ansprüche 1 bis 4, dadruch gekennzeichnet, dass er Kieselsäure oder Aluminiumoxid als poröses Trägermaterial enthält.

6. Verfahren zur Herstellung eines Trägerkatalysators gemäss Anspruch 1, dadurch gekennzeichnet, dass man

a) ein trockenes, poröses Trägermaterial bis zum Erreichen von 40 bis 80% des vorher ermittelten Sättigungswertes mit Wasser tränkt;

b) das vorgetränkte Trägermaterial nacheinander, jedoch in beliebiger Reihenfolge, mit einer ersten Lösung von wasserlöslichen Verbindungen des Chroms und gegebenenfalls des Kaliums in Wasser, einer zweiten Lösung einer wasserlöslichen Verbindung des Wolframs in Wasser und gegebenenfalls einer dritten Lösung einer wasserlöslichen Verbindung des Molybdäns in Wasser imprägniert, wobei jede Lösung mindestens einmal in einer höchstens zur vollständigen Sättigung des Trägermaterials ausreichenden Menge eingesetzt wird;

c) das Trägermaterial nach jeder Imprägnierung 2 bis 20 h bei 350 bis 500 K trocknet und

d) abschliessend 0,5 bis 12 h bei 550 bis 1000 K, vorzugsweise bei 600 bis 900 K, im Luftstrom sintert.

7. Verfahren nach Anspruch 6, dadurch gekennzeichnet, dass man das vorgetränkte Trägermaterial gemäss b) bei Temperaturen von 290 bis 375 K imprägniert.

8. Verfahren nach Anspruch 6 oder 7, dadurch gekennzeichnet, dass man das vorgetränkte Trägermaterial gemäss b) kontinuierlich unter teilweisem Eindampfen imprägniert.

9. Verwendung des Trägerkatalysators gemäss Anspruch 1 zur Herstellung von Styrol durch oxidative Dehydrierung von Ethylbenzol mit molekularem Sauerstoff in der Gasphase.

### Claims

1. Carrier-supported catalyst comprising oxides of chromium and tungsten and at least one of the oxides of molybdenum and potassium in the atomic ratio of $Cr_1W_{0.1-0.5}Mo_{0-0.05}K_{0-0.5}$ on a porous carrier material.

2. Carrier-supported catalyst as claimed in claim 1, wherein the carrier material has a BET-surface area of 0.1 to 500 $m^2/g$, preferably 2 to 200 $m^2/g$.

3. Carrier-supported catalyst as claimed in claim 1 or 2, wherein the carrier material consists of particles with a size of 0.01 to 6 mm, preferably 0.01 to 0.2 mm, for use in a flow bed, or of 3 to 6 mm for use in a fixed bed.

4. Carrier-supported catalyst as claimed in any of claims 1 to 3, containing 2 to 30 weight % of the oxides of chromium and tungsten as well as of molybdenum and/or potassium.

5. Carrier-supported catalyst as claimed in any of claims 1 to 4, containing silicic acid or aluminium oxide as the porous carrier material.

6. Process for making a carrier-supported catalyst as claimed in claim 1, which comprises:

a) impregnating a dry porous carrier material with water up to 40 to 80% of its predetermined saturation value;

b) impregnating the pre-impregnated carrier material successively, but in any desired sequential order, with a first solution of water-soluble compounds of chromium and, if desired, potassium in water with a second solution of a water-soluble compound of tungsten in water and, if desired, with a third solution of a water-soluble compound of molybdenum in water, each of these solutions being used at least once in a quantity which is at most necessary for complete saturation of the carrier material;

c) drying the carrier material after each impregnation over a period of 2 to 20 h at 350 to 500° K; and

d) sintering the carrier material over a period of 0.5 to 12 h at 550 to 1000° K, preferably 600 to 900° K, in a stream of air.

7. Process as claimed in claim 6, wherein the pre-impregnated carrier material is impregnated as described under b at temperatures of 290 to 375° K.

8. Process as claimed in claim 6 or 7, wherein the pre-impregnated carrier material is continuously impregnated as described under b with partial evaporation.

9. Use of the carrier-supported catalyst as claimed in claim 1 in the production of styrene by subjecting ethylbenzene to oxidative dehydrogenation with molecular oxygen in gas phase.

### Revendications

1. Catalyseur sur support constitué d'oxydes de chrome et de tungstène, et d'au moins un des oxydes de molybdène et de potassium dans des proportions atomiques de $Cr_1W_{0.1-0.5}Mo_{0-0.05}K_{0-0.5}$ sur une matière de support poreuse.

2. Catalyseur sur support selon la revendication 1, caractérisé en ce que la matière de support possède une surface BET de 0,1 à 500, de préférence de 2 à 200 $m^2/g$.

3. Catalyseur sur support selon l'une des revendications 1 ou 2, caractérisé en ce que la matière de support est à une dimension de grains de 0,01 à 6 mm, de préférence de 0,01 à 0,2 mm pour l'utilisation en lit fluidisé, ou de 3 à 6 mm pour l'utilisation en lit fixe.

4. Catalyseur sur support selon l'une des revendications 1 à 3, caractérisé en ce qu'il contient de 2 à 30% en poids des oxydes de chrome et de tungstène, de molybdène et/ou de potassium.

5. Catalyseur sur support selon l'une des revendications 1 à 4, caractérisé en ce qu'il contient de la silice ou de l'alumine en tant que matière de support poreuse.

6. Procédé de préparation d'un catalyseur sur support selon la revendication 1, caractérisé en ce que:

a) on imprègne une matière de support poreuse sèche par l'eau jusqu'à un niveau de 40 à 80% de la valeur de saturation déterminée au préalable;

b) on imprègne ensuite la matière de support successivement, mais dans un ordre quelconque, par une première solution de composés hydrosolubles de chrome et, le cas échéant, de potassium dans l'eau, une deuxième solution d'un composé hydrosoluble de tungstène dans l'eau et, le cas échéant, une troisième solution d'un composé hydrosoluble de molybdène dans l'eau, chacune des solutions étant utilisée au moins une fois en quantité au maximum suffisante pour saturer complètement la matière de support;

c) on sèche la matière de support pendant 2 à 20 h à une température de 350 à 500° K après chaque imprégnation, et

d) pour terminer, on fritte pendant ½ à 12 h à une température de 550 à 1000°K, de préférence de 600 à 900°K, dans un courant d'air.

7. Procédé selon la revendication 6, caractérisé en ce que, après imprégnation au préalable par l'eau, on imprègne la matière de support selon b à des températures de 290 à 375°K.

8. Procédé selon l'une des revendications 6 ou 7, caractérisé en ce que, après imprégnation de la matière de support par l'eau, on procède à l'imprégnation selon b en continu avec évaporation partielle.

9. Utilisation du catalyseur sur support selon la revendication 1 pour la préparation du styrène par déshydrogénation oxydante de l'éthylbenzène à l'aide d'oxygène moléculaire en phase gazeuse.